# EUROPÄISCHE PATENTSCHRIFT

(11) **EP 2 513 648 B1**
(45) Veröffentlichungstag und Bekanntmachung des Hinweises auf die Patenterteilung: **27.05.2015**
(21) Anmeldenummer: 10796365.4
(22) Anmeldetag: 15.12.2010
(51) Int. Cl.: G01N 33/50, C12Q 1/54

(54) **DETEKTION DER ZERSETZUNG VON ENZYMEN IN EINEM TESTELEMENT DURCH KONTROLLIERTE FREISETZUNG VON GESCHÜTZTEM ANALYT**
DETECTION OF THE DECOMPOSITION OF ENZYMES IN A TEST ELEMENT THROUGH CONTROLLED RELEASE OF PROTECTED ANALYTE
DÉTECTION DE LA DÉCOMPOSITION D'ENZYMES DANS UN ÉLÉMENT DE TEST PAR LIBÉRATION CONTRÔLÉE D'ANALYTE PROTÉGÉ

(30) Priorität: 16.12.2009 EP 09179500
(43) Veröffentlichungstag der Anmeldung: 24.10.2012
(73) Patentinhaber: F.Hoffmann-La Roche AG, 4070 Basel (CH); Roche Diagnostics GmbH, 68305 Mannheim (DE)
(72) Erfinder: HORN, Carina, 68647 Biblis (DE); HEINDL, Dieter, 82396 Paehl (DE); HAAR, Hans-Peter, 69168 Wiesloch (DE); STEINKE, Nelli, 68623 Lampertheim (DE)
(74) Vertreter: Weickmann & Weickmann
(86) Internationale Anmeldenummer: PCT/EP2010/069760
(87) Internationale Veröffentlichungsnummer: WO 2011/073258

(56) Entgegenhaltungen:
- EP-A1- 1 965 198
- EP-A2- 0 217 246
- WO-A1-2006/065900
- DE-U1- 29 620 452
- US-A1- 2004 126 831
- TUSA J K ET AL: "FLUORESCENT OPTICAL SENSORS FOR CRITICAL CARE ANALYTES", ANNALES DE BIOLOGIE CLINIQUE, JOHN LIBBEY EUROTEXT LTD, PARIS, FR, Bd. 61, Nr. 2, 1. März 2003 (2003-03-01), Seiten 183-191, XP008056604, ISSN: 0003-3898

## Beschreibung

Die vorliegende Erfindung betrifft ein diagnostisches Element zur Bestimmung mindestens eines Analyten sowie ein analytisches Messgerät, welches ein derartiges diagnostisches Element umfasst. Weiterhin betrifft die Erfindung ein Verfahren zur Bestimmung eines Analyten, ein Verfahren zur Berichtigung eines von einem Analyten erzeugten Signals sowie ein Verfahren zur Überprüfung der Detektionsoptik eines analytischen Messgeräts unter Verwendung des diagnostischen Elements. Schließlich betrifft die Erfindung ein System zur kontrollierten Freisetzung eines Reagenzes, sowie die Verwendung eines derartigen Systems als Schaltelement.

Diagnostische Elemente sind wichtige Bestandteile klinisch relevanter Analyseverfahren. Hierbei steht die Messung von Analyten, z. B. Metaboliten oder Substraten, im Vordergrund, welche beispielsweise mit Hilfe eines für den Analyten spezifischen Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym, einem Coenzym und gegenenfalls einem Mediator in Kontakt gebracht, wobei das Coenzym durch die enzymatische Reaktion physikochemisch verändert, z. B. oxidiert bzw. reduziert, wird. Sofern zusätzlich ein Mediator zum Einsatz gelangt, überträgt dieser die bei Umsetzung des Analyten freigesetzten Elektronen vom reduzierten Coenzym üblicherweise auf einen optischen Indikator oder die leitfähigen Bestandteile einer Elektrode, so dass der Vorgang beispielsweise photometrisch oder elektrochemisch erfasst werden kann. Eine Kalibrierung liefert einen direkten Zusammenhang des Messwerts mit der Konzentration des zu bestimmenden Analyten.

Aus dem Stand der Technik bekannte diagnostische Elemente zeichnen sich durch eine zeitlich begrenzte Haltbarkeit sowie durch spezielle Anforderungen an die Umgebung, wie Kühlung oder trockene Lagerung, zur Erzielung dieser Haltbarkeit aus. Bei bestimmten Anwendungsformen, z. B. bei Tests, die vom Endverbraucher selbst durchgeführt werden, wie etwa beim Blutglucose-Selbstmonitoring, können daher durch eine falsche, unbemerkte Fehllagerung des Messsystems Fehlergebnisse vorkommen, welche vom Verbraucher kaum zu erkennen sind und ggf. zu einer Fehlbehandlung der jeweiligen Erkrankung führen können.

Die Fehlergebnisse beruhen in erster Linie auf der Tatsache, dass die in derartigen diagnostischen Elementen eingesetzten Enzyme, Coenzyme und Mediatoren im Allgemeinen empfindlich auf Feuchtigkeit und Wärme reagieren und dabei inaktiviert werden. So nehmen beispielsweise bei der Detektion von Glucose mittels des Systems Glucose-Dehydrogenase/NAD unter feuchtwarmen Umgebungsbedingungen sowohl die Aktivität des Enzyms Glucose-Dehydrogenase als auch der Gehalt des Coenzyms NAD mit der Zeit ab, wobei die Abnahme an Coenzym im Allgemeinen deutlich schneller voranschreitet als der Aktivitätsverlust des Enzyms.

Eine bekannte Maßnahme, die zur Erhöhung der Stabilität von diagnostischen Elementen eingesetzt wird, ist die Verwendung stabiler Enzyme, z. B. die Verwendung von Enzymen aus thermophilen Organismen. Weiterhin besteht die Möglichkeit, Enzyme durch chemische Modifizierung, z. B. Quervernetzung, oder durch Mutagenese zu stabilisieren. Darüber hinaus können auch Enzymstabilisatoren, wie z. B. Trehalose, Polyvinylpyrrolidon und Serumalbumin, zugesetzt werden oder die Enzyme z. B. durch Photopolymerisation in Polymernetzwerke eingeschlossen werden.

Eine weitere Möglichkeit, die Haltbarkeit diagnostischer Elemente zu verbessern, besteht in der Verwendung stabilisierter Coenzyme. Während native Coenzyme, wie beispielsweise NAD und NADP bzw. ihre reduzierten Formen NADH und NADPH, unter basischen bzw. sauren Bedingungen aufgrund der Labilität der Glykosyl-Bindung zwischen der Ribose- und der Pyridineinheit vergleichsweise instabil sind, wurden in der Literatur in den vergangenen Jahren verschiedene Derivate von NAD/NADH und NADP/NADPH beschrieben, welche durch Modifizierung der Nikotinamidgruppe bzw. der Ribose-Einheit die Stabilität des Coenzyms signifikant erhöhen.

Schließlich kann eine Erhöhung der Stabilität diagnostischer Elemente auch durch Verwendung stabiler Mediatoren erzielt werden. So wird durch die Verwendung von Mediatoren mit möglichst niedrigem Redoxpotential die Spezifität von Tests erhöht und Störungen während der Reaktion eliminiert. Eine untere Grenze für das Redoxpotential von Mediatoren bilden jedoch die Redoxpotentiale der Enzym/Coenzym-Komplexe. Werden diese unterschritten, wird die Reaktion mit den Mediatoren verlangsamt oder gar unterbunden.

Tatsächlich weisen die in diagnostischen Elementen verwendeten Komponenten chemischer Nachweisreagenzien in der Praxis allerdings nicht allesamt die gleiche Stabilität auf, sondern unterliegen vielmehr unterschiedlich schnell ablaufenden Zersetzungsprozessen. So tritt beispielsweise bei Verwendung von carba-NAD als Coenzym der Effekt auf, dass das Coenzym aufgrund der carbazyklischen Zuckereinheit auch bei Feuchtigkeit und erhöhten Temperaturen über lange Zeit sehr stabil bleibt, während die Aktivität eines in dem diagnostischen Element eingesetzten nativen Enzyms kontinuierlich abnimmt. Gleichermaßen kann beispielsweise im Falle der Verwendung einer Kombination aus stabilisiertem Enzym und nativem Coenzym die Aktivität des Enzyms über einen längeren Zeitraum aufrecht erhalten werden, während sich die Menge an Coenzym infolge thermischer oder/und hydrolytischer Zersetzung mehr oder weniger rasch reduziert.

Um Fehlergebnisse bei der Bestimmung von Analyten zu vermeiden, müssten diagnostische Elemente somit eine Feststellung ermöglichen, ob die einzelnen Komponenten eines in dem diagnostischen Element eingesetzten Nachweisreagenzes auch im Messzeitpunkt noch in funktioneller Form vorliegen und das Nachweisreagenz insofern zum qualitativen oder/und quantitativen Nachweis des Analyten geeignet ist. Als besonders problematisch gestaltet sich in diesem Zusammenhang beispielsweise die Bestimmung der Enzymaktivität, da die Inaktivierung eines Enzyms zunächst durch eine Konformationsänderung des Proteins hervorgerufen wird und somit mangels Änderung der Konstitution keine optisch- oder elektrochemisch-aktiven Teilchen gebildet werden.

Die der vorliegenden Erfindung zugrunde liegende Aufgabe bestand somit darin, ein stabiles diagnostisches Element insbesondere zur Bestimmung von Glucose bereitzustellen, bei welchem die Nachteile des Standes der Technik zumindest teilweise beseitigt sind. Insbesondere sollte das diagnostische Element gewährleisten, dass im Falle einer stark verminderten oder gänzlich fehlenden Funktionsfähigkeit einzelner Komponenten eines Nachweisreagenzes die Angabe vermeintlich korrekter Messergebnisse in Bezug auf einen zu bestimmenden Analyten vermieden wird.

Diese Aufgabe wird erfindungsgemäß gelöst durch ein diagnostisches Element zur Bestimmung mindestens eines Analyten, umfassend ein für den Analyten spezifisches Nachweisreagenz und eine definierte Menge des zu bestimmenden Analyten (Indikatoranalyt), wobei der Indikatoranalyt in einer für eine Reaktion mit dem Nachweisreagenz unzugänglichen, freisetzbaren Form vorliegt.

Das in dem erfindungsgemäßen diagnostischen Element eingesetzte Nachweisreagenz ist vorzugsweise ein chemisches Nachweisreagenz und kann beliebige Komponenten umfassen, welche zur Bestimmung eines Analyten, beispielsweise unter Verwendung optischer oder elektrochemischer Mittel, geeignet sind. Beispiele für derartige Komponenten sind dem Fachmann bekannt und umfassen insbesondere Enyzme, Coenzyme, Mediatoren, optische Indikatoren sowie Hilfs- oder/und Zusatzstoffe, sind jedoch nicht auf diese beschränkt.

In einer bevorzugten Ausführungsform umfasst das Nachweisreagenz mindestens ein Enzym oder/und Coenzym, welche unabhängig voneinander natürlichen, semisynthetischen oder synthetischen Ursprungs sein können und vom Fachmann je nach Anforderung an das diagnostische Element frei wählbar sind. Besonders bevorzugt ist erfindungsgemäß eine Kombination aus mindestens einem Enzym und mindestens einem Coenzym, wobei das Enzym vorzugsweise eine hohe Spezifität für den zu bestimmenden Analyten aufweist und damit Fehlergebnisse im Rahmen der Bestimmung des Analyten minimiert.

Sofern das erfindungsgemäße diagnostische Element ein Enzym umfasst, handelt es sich bei dem Enzym vorzugsweise um ein Coenzym-abhängiges Enzym. Beispiele für derartige Enzyme umfassen u.a. Dehydrogenasen, Oxidasen, wie z. B. Glucoseoxidase (EC 1.1.3.4) oder Cholesterinoxidase (EC 1.1.3.6), Aminotransferasen, wie z. B. Aspartat- oder Alanin-Aminotransferase, 5'-Nukleotidase, Creatin-Kinase und Diaphorase (EC 1.6.99.2). In einer stärker bevorzugten Ausführungsform gelangt als Enzym eine Nikotinamid-Adenin-Dinukleotid (NAD/NADH)- oder Nikotinamid-Adenin-Dinukleotidphosphat (NADP/NADPH)-abhängige Dehydrogenase zur Anwendung, wobei das Enzym insbesondere aus der Gruppe bestehend aus einer Alkohol-Dehydrogenase (EC 1.1.1.1; EC 1.1.1.2), einer L-Aminosäure-Dehydrogenase (EC 1.4.1.5), einer Glucose-Dehydrogenase (EC 1.1.1.47), einer Glucose-6-phosphat-Dehydrogenase (EC 1.1.1.49), einer Glycerin-Dehydrogenase (E.C.1.1.1.6), einer 3-Hydroxybutyrat-Dehydrogenase (EC 1.1.1.30), einer Lactat-Dehydrogenase (EC 1.1.1.27; 1.1.1.28), einer Malat-Dehydrogenase (EC 1.1.1.37) und einer Sorbitol-Dehydrogenase ausgewählt wird. Am stärksten bevorzugt ist das Enzym eine Glucose-Dehydrogenase (EC 1.1.1.47) oder eine Glucose-6-phosphat-Dehydrogenase (EC 1.1.1.49).

Wird als Enzym eine Glucose-Dehydrogenase (EC 1.1.1.47) verwendet, so kann im Rahmen des erfindungsgemäßen Verfahrens beispielsweise eine mutierte Glucose-Dehydrogenase zur Anwendung gelangen. Der Begriff "Mutante", wie er im Rahmen der vorliegenden Anmeldung verwendet wird, bezeichnet eine genetisch veränderte Variante eines nativen Enzyms, welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Die Einführung der Mutation(en) kann ortsspezifisch oder nicht-ortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf. Beispiele für derartige Mutanten werden von Baik (Appl. Environ. Microbiol. (2005), 71, 3285), Väsquez-Figueroa (ChemBioChem (2007), 8, 2295) sowie in WO 2005/045016 A2 beschrieben, auf deren Offenbarung hiermit ausdrücklich Bezug genommen wird.

Die mutierte Glucose-Dehydrogenase kann die gegenüber der korrespondierenden Wildtyp-Glucose-Dehydrogenase veränderte(n) Aminosäure(n) grundsätzlich an einer beliebigen Position ihrer Aminosäuresequenz enthalten. Vorzugsweise umfasst die mutierte Glucose-Dehydrogenase eine Mutation an mindestens einer der Positionen 96, 170 und 252 der Aminosäuresequenz der Wildtyp-Glucose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z. B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glucose-Dehydrogenase kann durch Mutation einer aus einer beliebigen biologischen Quelle stammenden Wildtyp-Glucose-Dehydrogenase erhalten werden, wobei der Begriff "biologische Quelle" im Sinne dieser Erfindung sowohl Prokaryoten, wie beispielsweise Bakterien, als auch Eukaryoten, wie beispielsweise Säuger und andere Tiere, umfasst. Vorzugsweise entstammt die Wildtyp-Glucose-Dehydrogenase einem Bakterium, wobei es sich besonders bevorzugt um eine Glucose-Dehydrogenase aus Bacillus megaterium, Bacillus subtilis oder Bacillus thuringiensis, insbesondere aus Bacillus subtilis, handelt.

In einer besonders bevorzugten Ausführungsform handelt es sich bei der mutierten Glucose-Dehydrogenase um eine durch Mutation von Wildtyp-Glucose-Dehydrogenase aus Bacillus subtilis erhaltene Glucose-Dehydrogenase, welche die in SEQ ID NO: 1 (GlucDH_E96G_E170K) oder die in SEQ ID NO: 2 (GlucDH_E170K_K252L) dargestellte Aminosäuresequenz besitzt.

In einer Ausführungsform der Erfindung umfassen die hierin beschriebenen diagnostischen Elemente weiterhin ein Coenzym, welches vorzugsweise ein stabilisiertes Coenzym ist. Ein stabilisiertes Coenzym im Sinne der vorliegenden Erfindung ist ein gegenüber dem nativen Coenzym chemisch verändertes Coenzym, welches bei Atmosphärendruck eine im Vergleich zum nativen Coenzym höhere Stabilität gegenüber Feuchtigkeit,

Temperaturen insbesondere im Bereich von 0°C bis 50°C, Säuren und Basen insbesondere im Bereich von pH 4 bis pH 10, oder/und Nukleophilen wie beispielsweise Alkoholen oder Aminen, aufweist und insofern unter identischen Umgebungsbedingungen über einen längeren Zeitraum als das native Coenzym seine Wirkung entfalten kann. Vorzugsweise weist das stabilisierte Coenzym eine im Vergleich zum nativen Coenzym höhere hydrolytische Stabilität auf, wobei eine vollständige Hydrolysestabilität unter Testbedingungen besonders bevorzugt ist. Im Vergleich zum nativen Coenzym kann das stabilisierte Coenzym eine verringerte Bindungskonstante für das Enzym aufweisen, beispielsweise eine um den Faktor von 2 oder mehr verringerte Bindungskonstante.

Bevorzugte Beispiele für stabilisierte Coenzyme im Sinne der vorliegenden Erfindung sind stabilisierte NAD(P)/NAD(P)H-Verbindungen, d. h. chemische Derivate von nativem Nikotinamid-Adenin-Dinukleotid (NAD/NADH) bzw. Nikotinamid-Adenin-Dinukleotidphosphat (NADP/NADPH), oder die Verbindung der Formel (I)

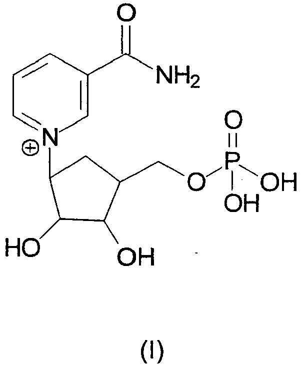

Sofern es sich bei dem stabilisierten Coenzym um eine stabilisierte NAD(P)/NAD(P)H-Verbindung handelt, umfasst die stabilisierte NAD(P)/NAD(P)H-Verbindung vorzugsweise einen 3-Pyridincarbonyl- oder einen 3-Pyridinthiocarbonyl-Rest, welcher ohne glykosidische Bindung über einen linearen oder cyclischen organischen Rest, insbesondere über einen cyclischen organischen Rest, mit einem phosphorhaltigen Rest, wie beispielsweise einem Phosphatrest, verknüpft ist.

Besonders bevorzugt wird die stabilisierte NAD(P)/NAD(P)H-Verbindung aus Verbindungen der allgemeinen Formel (II) ausgewählt: 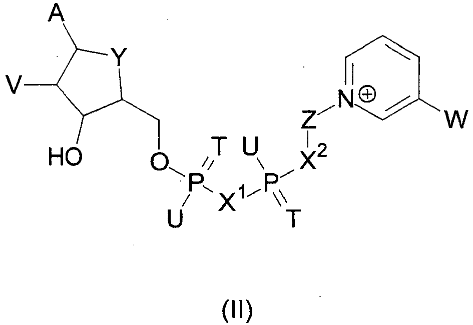 worin
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

In einer bevorzugten Ausführungsform enthalten die Verbindungen der allgemeinen Formel (II) Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁₋₆-Alkinyl, -Alkenyl oder -Alkyl substituiert sein können.

In einer weiteren bevorzugten Ausführungsform enthalten die Verbindungen der allgemeinen Formel (II) Adenosin-Analoga, welche statt Ribose z.B. 2-Methoxydesoxyribose, 2'-Fluorodesoxyribose, Hexitol, Altritol bzw. polycyclische Analoga, wie Bicyclo-, LNA- und Tricyclo-Zucker enthalten.

Insbesondere können in den Verbindungen der allgemeinen Formel (II) auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S. In den erfindungsgemäßen Verbindungen der Formel (II) ist W vorzugsweise CONH₂ oder COCH₃.

In den Verbindungen der allgemeinen Formel (II) ist Z vorzugsweise ein linearer Rest mit 4-6 C-Atomen, vorzugsweise mit 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättiger oder ungesättigter carbocyclischer oder heterocyclischer Fünfring, insbesondere eine Gruppe der allgemeinen Formel (III), 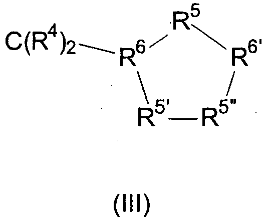 wobei zwischen R⁵' und R⁵" eine Einfach- oder Doppelbindung vorliegen kann, mit
- R⁴ =: jeweils unabhängig H, F, Cl, CH₃,
- R⁵ =: CR⁴₂,
- R⁵' =: O, S, NH, NC₁-C₂-Alkyl, CR⁴₂, CHOH, CHOCH₃, und R⁵" = CR⁴₂, CHOH, CHOCH₃, wenn zwischen R⁵' und R⁵" eine Einfachbindung vorliegt,
- R⁵' = R⁵" = CR⁴,: wenn zwischen R⁵' und R⁵" eine Doppelbindung vorliegt, und
- R⁶, R⁶' =: jeweils unabhängig CH oder CCH₃.

In den Gruppen der allgemeinen Formel (III) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂. Am stärksten bevorzugt ist eine Gruppe der Formel (III), in welcher R⁴ = H ist, R⁵ = CH₂ ist, R⁵' = R⁵" = CHOH ist, und R⁶ = R⁶' = CH ist.

In der am stärksten bevorzugten Ausführungsform handelt es sich bei dem stabilisierten Coenzym um carbaNAD (J.T. Slama, Biochemistry (1988), 27, 183 und Biochemistry (1989), 28, 7688) oder um carbaNADP. Andere stabile Coenzyme, welche erfindungsgemäß Anwendung finden können, sind in WO 98/33936, WO 01/49247, WO 2007/012494, US 5,801,006, US 11/460,366 und der Publikation Blackburn et al. (Chem. Comm. (1996), 2765), beschrieben, auf deren Offenbarung hiermit ausdrücklich Bezug genommen wird.

In einer weiteren bevorzugten Ausführungsform der Erfindung umfasst das Nachweisreagenz mindestens eine weitere Komponente, welche dem qualtitativen Nachweis oder/und der quantitativen Bestimmung des Analyten dient, wie etwa einen Mediator oder/und einen optischen Indikator. Der Begriff "Mediator", wie er im Rahmen dieser Anmeldung verwendet wird, bezeichnet eine chemische Verbindung, welche die Reaktivität des durch Umsetzung mit dem Analyten erhaltenen, reduzierten Coenzyms erhöht und eine Übertragung von Elektronen auf einen geeigneten optischen Indikator oder ein optisches Indikatorsystem ermöglicht. Als Mediatoren kommen erfindungsgemäß u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenaziniumtrifluormethansulfonat, oder/und Diaphorase (EC 1.6.99.2) in Frage.

Bevorzugte Beispiele für Phenanthrolinchinone im Sinne der vorliegenden Erfindung umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind. Diaphorasen, welche für die Zwecke der vorliegenden Erfindung besonders geeignet sind, umfassen beispielsweise Diaphorase aus Schweineherz, Clostridium kluyverii und Bacillus stearothermophilus sowie die in US 2007/0196899 A1 beschriebene Diaphorase-Mutante, welche eine gegenüber nativen Diaphorasen verbesserte katalytische Funktion und Thermostabilität aufweist. Auf die Offenbarung obiger US-Anmeldung wird hiermit ausdrücklich Bezug genommen.

Als optischer Indikator kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden optischen, insbesondere photometrischen oder fluorimetrischen, oder elektrochemischen Verfahrens erfolgen.

Vorzugsweise werden Heteropolysäuren, und insbesondere 2,18-Phosphormolybdänsäure, als optische Indikatoren verwendet, die zum entsprechenden Heteropolyblau reduziert werden. Alternativ können auch Chinone wie beispielsweise Resazurin, Dichlorphenolindophenol oder/und Tetrazoliumsalze als optische Indikatoren eingesetzt werden. Tetrazoliumsalze, welche für die Zwecke der vorliegenden Erfindung besonders geeignet sind, umfassen beispielsweise die kommerziell erhältlichen Produkte WST-3, WST-4 und WST-5 (alle Fa. Dojindo), sind jedoch nicht auf diese beschränkt.

Erfindungsgemäß enthalten die hierin beschriebenen diagnostischen Elemente weiterhin eine definierte Menge des zu bestimmenden Analyten (Indikatoranalyt), welche der Erzeugung eines definierten, optisch oder elektrochemisch detektierbaren Signals dient. Entspricht das durch Reaktion des Indikatoranalyten mit dem Nachweisreagenz erzeugte Signal in seiner Größe einem vorgegebenen Signal (Referenzsignal), welches mit einer ausreichenden Menge an den zur Bestimmung des Analyten benötigten, in funktioneller Form vorliegenden Einzelkomponenten des Nachweisreagenzes (z. B. mit einer ausreichenden Menge an aktivem Enzym) korreliert, so gibt das diagnostische Element das Ergebnis der eigentlichen Messung des Probenanalyten frei.

Der Begriff "in funktioneller Form", wie er hierin verwendet wird, bedeutet, dass die betreffende Komponente des Nachweisreagenzes in chemisch aktiver Form vorliegt und die ihr zugedachte Funktion in dem diagnostischen Element erfüllen kann. Der Begriff "in nicht-funktioneller Form" bedeutet demgegenüber, dass die Komponente in chemisch inaktiver Form vorliegt oder ihre vorgesehene Funktion trotz Vorliegens in einer chemisch aktiven Form, welche sich von der zur Ausübung der gewünschten Funktion erforderlichen Form unterscheidet, nicht erfüllt.

Das im Rahmen der vorliegenden Anmeldung beschriebene diagnostische Element kann ein beliebiges Testelement sein, welches eine das Nachweisreagenz enthaltende trockene Reagenzienschicht umfasst und von der den Analyten enthaltenden Probe benetzt werden kann. Vorzugsweise umfassen die erfindungsgemäßen Testelemente eine Trägerschicht sowie eine das Nachweisreagenz enthaltende Nachweisschicht. Die Trägerschicht kann hierbei aus einem beliebigen Material gebildet sein, auf welches die Nachweisschicht unter Verwendung geeigneter Techniken aufgebracht werden kann und welches in der Folge als Träger für das zur Bestimmung des Analyten verwendete Nachweiseragenz fungiert. Das Nachweisreagenz kann neben Enzym, Coenzym, Mediator oder/und optischem Indikator ggf. weitere Reagenzien umfassen, welche dem Fachmann für die Zwecke der jeweiligen Anwendung geeignet erscheinen oder/und zur Herstellung diagnostischer Elemente üblicherweise benötigt werden, wie beispielsweise Hilfs- oder/und Zusatzstoffe.

Die räumliche Positionierung des Indikatoranalyten innerhalb der diagnostischen Elemente umfasst verschiedene Varianten, welche von einem Fachmann entsprechend den jeweiligen Anforderungen und Wünschen ausgewählt werden können. In einer bevorzugten Ausführungsform enthalten die erfindungsgemäßen Testelemente den Indikatoranalyten in der Nachweisschicht, so dass das Nachweisreagenz und der Indikatoranalyt in einer gemeinsamen Schicht vorliegen. In einer anderen bevorzugten Ausführungsform der Erfindung ist der Indikatoranalyt demgegenüber in einer von der Nachweisschicht separaten Depotschicht, insbesondere in einer zwischen der Trägerschicht und der Nachweisschicht befindlichen Depotschicht, angeordnet, wobei die Depotschicht in direktem Kontakt mit der Nachweisschicht stehen oder von dieser durch eine oder mehrere weitere Schichten abgetrennt sein kann. Besonders bevorzugt ist zwischen einer solchen Depotschicht und der Nachweisschicht eine auflösbare, insbesondere photochemisch oder elektrochemisch auflösbare Sperrschicht angeordnet, welche ihrerseits vorzugsweise in direktem Kontakt mit der Nachweisschicht steht.

Um eine unkontrollierte Reaktion mit dem Nachweisreagenz des diagnostischen Elements und damit eine Verfälschung der eigentlichen Analytenmessung zu vermeiden, enthalten die erfindungsgemäßen Testelemente den Indikatoranalyten in einer für eine Reaktion mit dem Nachweisreagenz unzugänglichen, freisetzbaren Form. Der Ausdruck "in einer für eine Reaktion mit dem Nachweisreagenz unzugänglichen, freisetzbaren Form", wie er in vorliegender Anmeldung verwendet wird, ist als synonym mit dem Ausdruck "in geschützter Form" anzusehen und umfasst jegliche Möglichkeit, eine (vorzeitige) Reaktion zwischen dem Indikatoranalyten und dem Nachweisreagenz gezielt zu verhindern. Maßnahmen zum Schutz des Indikatoranalyten vor einer unerwünschten chemischen Umsetzung umfassen u.a. eine chemische Derivatisierung des Indikatoranalyten unter Verwendung geeigneter Schutzgruppen, eine Verkapselung des Indikatoranalyten in einer auflösbaren und gegenüber dem Nachweisreagenz inerten Matrix, sowie eine räumliche Abtrennung des Indikatoranalyten von der das Nachweisreagenz enthaltenden Nachweisschicht oder/und Depotschicht.

Während der Indikatoranalyt im Falle einer chemischen Derivatisierung aufgrund der hierdurch bewirkten Inaktivierung in direktem Kontakt mit dem Nachweisreagenz des diagnostischen Elements stehen kann, muss im Falle der Verwendung eines nicht mit chemischen Schutzgruppen versehenen Indikatoranalyten ein direkter Kontakt mit dem Nachweisreagenz vermieden werden. Zu diesem Zweck kann der Indikatoranalyt beispielsweise in einer organischen oder anorganischen, gegenüber dem Nachweisreagenz inerten Matrix verkapselt sein, welche unter definierten Bedingungen auflösbar ist und den Indikatoranalyten freisetzt. Alternativ kann der Indikatoranalyt, um eine vorzeitige Reaktion mit dem Nachweisreagenz zu vermeiden, durch mindestens eine unter definierten Bedingungen auflösbare Sperrschicht von der Nachweisschicht abgetrennt sein, wodurch eine Freisetzung des Indikatoranalyten beispielsweise aus der Depotschicht in die Nachweisschicht ermöglicht wird.

Um eine chemische Umsetzung mit dem Nachweisreagenz zu ermöglichen, muss der Indikatoranalyt in dem diagnostischen Element zu gegebener Zeit aus seiner für eine Reaktion mit dem Nachweisreagenz unzugänglichen Form freigesetzt werden. Die Freisetzung kann unter Verwendung geeigneter Mittel, insbesondere photochemisch durch Bestrahlen mit Licht geeigneter Wellenlänge oder elektrochemisch durch Anlegen einer Spannung, und zu einem beliebigen Zeitpunkt, insbesondere vor oder nach Reaktion des Nachweisreagenzes mit dem Probenanalyten, erfolgen, wobei eine ggf. vorhandene Schutzgruppe des Indikatoranalyten abgespalten, eine den Indikatoranalyten enthaltende Matrix zumindest teilweise aufgelöst, oder/und eine zwischen der Depotschicht und der Nachweisschicht angeordnete, auflösbare Sperrschicht zumindest teilweise zerstört wird. Die teilweise oder vollständige Zerstörung der auflösbaren Matrix oder Sperrschicht kann beispielsweise unter Verwendung der Methode des spektralen Lochbrennens ("hole-burning") erfolgen, welche auf kristalline und amorphe Festkörper angewendet werden kann und insbesondere in der optischen Informationsverarbeitung, Molekularelektronik, integrierten Optik und Optoelektronik genutzt wird (C. Bräuchle, Angewandte Chemie (1992), 104(4), 431-435).

Für die Anwendung der Methode des spektralen Lochbrennens im Rahmen der vorliegenden Erfindung sind polymere Matrices mit darin enthaltenen Energieabsorbern von besonderem Interesse. Die Energieabsorber absorbieren gemäß ihrem Absorptionsspektrum Energie und geben diese, sofern keine anderen Wege wie beispielsweise Fluoreszenzstrahlung zum Energieabbau zur Verfügung stehen, an ihre lokale Umgebung ab. Je nach Dauer und Intensität der Bestrahlung dehnt sich die umgebende Polymermatrix infolge Erwärmung aus, schmilzt, platzt auf oder/und geht chemische Veränderungsreaktionen ein. Insofern kann eine mindestens einen Energieabsorber umfassende Polymermatrix beispielsweise zur Verkapselung des Indikatoranalyten oder/und als Sperrschicht verwendet werden, welche den Indikatoranalyten vor einer unerwünschten Reaktion mit dem Nachweisreagenz des diagnostischen Elements schützt.

Bei Zerstörung bzw. Generierung von Löchern in der Polymermatrix durch Bestrahlen mit Licht geeigneter Wellenlänge, insbesondere einer von der Wellenlänge der Analytenmessung um mindestens 50 nm differierenden Wellenlänge, kann der Indikatoranalyt unter definierten Bedingungen und zu einem beliebigen Zeitpunkt mit dem Nachweisreagenz in Kontakt gebracht werden, wobei vorzugsweise ein optisch oder elektrochemisch messbares Signal erzeugt wird. Sofern die Messung des Analyten beispielsweise im ultravioletten Bereich (d.h. bei einer Wellenlänge im Bereich unterhalb 400 nm), insbesondere bei einer Wellenlänge im Bereich von etwa 150 nm bis etwa 400 nm, erfolgt, kann zur Anregung des Energieabsorbers insofern beispielsweise Licht mit einer Wellenlänge von > 400 nm, insbesondere Licht mit einer Wellenlänge im Bereich von etwa 450 nm bis etwa 800 nm, eingestrahlt werden.

Insofern ermöglicht oben beschriebenes Verfahren allgemein die Bereitstellung von Systemen zur kontrollierten Freisetzung von Reagenzien, in welchen die Freisetzung unabhängig von anderen Reaktionsabläufen in dem System vorgenommen werden kann und insofern eine gezielte Steuerung von Reaktionsabläufen in dem System erlaubt. Folglich kann ein System, welches einen Träger, umfassend ein in geschützter Form vorliegendes und unter definierten Bedingungen freisetzbares Reagenz und ein davon separates Reaktionssystem, sowie Mittel zur Freisetzung des Reagenzes auf dem Träger umfasst, beispielsweise als Schaltelement insbesondere im Rahmen von Reaktionskaskaden eingesetzt werden. Der Schutz des Reagenzes vor Umsetzung mit dem Reaktionssystem kann hierbei insbesondere durch Verkapselung des Reagenzes in einer auflösbaren Matrix, durch räumliche Abtrennung des Reagenzes von dem Reaktionssystem mittels einer auflösbaren Sperrschicht, oder/und durch Verwendung chemischer Schutzgruppen bewirkt werden, wie im Rahmen der vorliegenden Anmeldung detailliert beschrieben.

In einer bevorzugten Ausführungsform umfasst die Nachweisschicht, die Sperrschicht oder/und die Depotschicht der erfindungsgemäßen Testelemente insofern eine Polymermatrix, welche mindestens einen Energieabsorber enthält, wodurch der Indikatoranalyt vor Bestrahlen der diagnostischen Elemente mit Licht geeigneter Wellenlänge, wie vorstehend definiert, vor einem direkten Kontakt mit dem Nachweisreagenz geschützt werden kann. Erfindungsgemäß kann die den Energieabsorber enthaltende Polymermatrix insbesondere in die Nachweisschicht der hierin beschriebenen diagnostischen Elemente eingebettet oder in Form einer separaten Schicht, beispielsweise als Sperrschicht, ausgebildet sein. Die Dicke einer solchen Sperrschicht liegt erfindungsgemäß im Bereich von 0.05 µm bis 5 µm, bevorzugt im Bereich von 1 µm bis 3 µm. Sofern die Polymermatrix nicht als Sperrschicht zum Zwecke einer räumlichen Abtrennung beispielsweise eines Indikatoranalyten, der keine chemischen Schutzgruppen aufweist, von der Nachweisschicht fungiert, so enthält die Polymermatrix neben dem Energieabsorber vorzugsweise auch den Indikatoranalyten, wobei der Indikatoranalyt in einer besonders bevorzugten Variante der Erfindung in der Polymermatrix verkapselt ist.

Die Polymermatrix ist bevorzugt aus mindestens einem hydrophoben Polymer gebildet, welches verhindert, dass der Indikatoranalyt im Falle eines Kontakts des diagnostischen Elements mit Feuchtigkeit Anlöseerscheinungen zeigt. Insbesondere handelt es sich bei dem Polymer um ein hydrophobes organisches Polymer, welches eine thermische Lochbildung begünstigt und bei Temperaturen von 5°C bis 50°C stabil ist, d. h. keinerlei Zersetzungserscheinungen zeigt. Als Polymer, welches zur Bildung einer Polymermatrix im Sinne der vorliegenden Erfindung geeignet ist, kommt prinzipiell jedes hydrophobe Polymer in Betracht, das nicht in bzw. durch Wasser gelöst werden kann. Besonders bevorzugt ist die oben beschriebene Polymermatrix aus einem hydrophoben Polymer ausgewählt aus der Gruppe bestehend aus einem Polymethylmethacrylat (PMMA), einem Polyethylmethacrylat (PEMA), einem Polycarbonat und chemischen Derivaten hiervon gebildet, obwohl auch andere dem Fachmann bekannte hydrophobe Polymere eingesetzt werden können.

Erfindungsgemäß kann die Polymermatrix den mindestens einen Energieabsorber in unterschiedlicher Form enthalten, beispielsweise kovalent gebunden oder/und in freier Form, wobei eine kovalente Bindung des Energieabsorbers innerhalb der Polymermatrix als bevorzugt angesehen wird. Enthält die Polymermatrix den Energieabsorber kovalent gebunden, so ist der Energieabsorber vorzugsweise unmittelbarer Bestandteil des hydrophoben Polymers und besonders bevorzugt in die Polymerkette des hydrophoben Polymers integriert, was beispielsweise durch Polymerisieren geeigneter Monomere des Energieabsorbers bewirkt werden kann. Auf diese Weise kann sichergestellt werden, dass unabhängig von der Löslichkeit des ausgewählten Energieabsorbers in einer zur Herstellung der Polymermatrix verwendeten Polymerlösung eine große Menge an Molekülen des Energieabsorbers in der erhaltenen Polymermatrix vorhanden ist, was eine hohe Energieaufnahme ermöglicht und insofern eine Auflösung der Polymermatrix zum Zwecke einer Freisetzung des Indikatoranalyten begünstigt. Soll der Energieabsorber in der Polymermatrix hingegen in freier Form bzw. ungebunden vorliegen, d. h. eine mit dem Energieabsorber dotierte Polymermatrix erzeugt werden, so kann dies beispielsweise durch einfaches Mischen von geeignetem Polymervorläufer und Energieabsorber sowie anschließendes Polymerisieren des Gemisches bewerkstelligt werden.

Als Energieabsorber, welcher in der Polymermatrix enthalten ist, kann entsprechend den jeweiligen Anforderungen ein beliebiger Energieabsorber verwendet werden, welcher die durch Bestrahlung aufgenommene Energie zumindest teilweise an seine lokale Umgebung abgibt und damit eine Veränderung der Polymermatrix bewirkt. Zur Unterstützung der Lochbildung kann die Polymermatrix oder/und das hydrophobe Polymer neben dem mindestens einen Energieabsorber weiterhin thermisch instabile Verbindungen bzw. thermisch instabile funktionelle Gruppen umfassen, welche bei entsprechender Wärmezufuhr leichtflüchtige Verbindungen, wie beispielsweise Stickstoff, Kohlendioxid oder andere bei Raumtemperatur gasförmige Verbindungen, freisetzen. Beispiele für derartige Verbindungen bzw. funktionelle Gruppen umfassen insbesondere Azoderivate, Kohlensäurederivate und cyclische Alkene, sind jedoch nicht auf diese beschränkt.

Bei dem erfindungsgemäß verwendeten Energieabsorber handelt es sich bevorzugt um einen hydrophoben Farbstoff, welcher vorzugsweise eine hohe Löslichkeit in der Polymermatrix aufweist oder/und nach Anregung keinerlei bzw. lediglich geringe Mengen an Fluoreszenzstrahlung emittiert und insofern eine geringe Quantenausbeute, insbesondere eine Quantenausbeute von null, besitzt. Stärker bevorzugt werden im Rahmen der Erfindung hydrophobe Farbstoffe eingesetzt, welche bei der Wellenlänge der eigentlichen Analytenmessung (beispielsweise bei λ = 375 nm) keine oder lediglich eine geringe Absorption zeigen, jedoch bei orthogonalen Wellenlängen (z. B. bei λ > 500 nm) einen hohen Extinktionskoeffizienten besitzen, wodurch die Gefahr einer unerwünschten Freisetzung des Indikatoranalyten während der eigentlichen Messung vermieden wird. Als besonders geeignet haben sich in diesem Zusammenhang Cyaninfarbstoffe, wie z.B. Cryptocyanin, Indotricarbocyanin (C7), Oxacarbocyanin (C3), Pinacyanoliodid, Thiacarbocyanin (C3), Thiadicarbocyanin (C5), oder Squarylium-Farbstoffe, z.B. Squarylium-Farbstoff III, erwiesen.

In einer weiteren Variante der Erfindung umfasst der in einer für eine Reaktion mit dem Nachweisreagenz unzugänglichen, freisetzbaren Form vorliegende Indikatoranalyt eine photochemisch oder/und elektrochemisch spaltbare Schutzgruppe, welche bei Bedarf abgespalten werden kann und im Zuge dessen die Freisetzung des Indikatoranalyten unter definierten Bedingungen ermöglicht. Beispiele für photochemisch spaltbare Schutzgruppen, welche beispielsweise zur Derivatisierung von Glucose verwendet werden können, umfassen insbesondere Anthrachinonderivate, Benzoesäurederivate, Coumarinderivate, Nitrobenzophenonderivate, Thioxanthenderivate, Thioxanthenonderivate, Xanthenderivate und Veratrylderivate, sind jedoch nicht auf diese beschränkt. Beispiele für elektrochemisch spaltbare Schutzgruppen, welche im Rahmen der vorliegenden Erfindung Anwendung finden können, umfassen u.a. Anthronderivate. Eine umfassende Übersicht über Schutzgruppen in der organischen Synthese findet sich in T.W. Greene, "Protecting Groups in Organic Synthesis", 2. Auflage, John Wiley and Sons, New York, 1991.

Die Freisetzung des Indikatoranalyten erfolgt erfindungsgemäß vorzugsweise vor oder nach Reaktion des Nachweisreagenzes mit dem Probenanalyten. Während unmittelbar (z. B. innerhalb der ersten Sekunde) nach Benetzung des diagnostischen Elements mit der Probe des Analyten noch keine Reaktion des Nachweisreagenzes mit dem zu bestimmenden Analyten erfolgt und damit eine Reaktion mit dem in definierter Menge vorliegenden Indikatoranalyten störungsfrei verfolgt werden kann, beobachtet man etwa eine Sekunde nach Benetzung des diagnostischen Elements die beginnende Umsetzung des in der Probe gelösten Probenanalyten. Insofern ist ab Eintritt der Umsetzung des Probenanalyten bis hin zum Zeitpunkt der nahezu vollständigen Umsetzung des Probenanalyten eine Freisetzung des Indikatoranalyten in dem diagnostischen Element unerwünscht, da eine Unterscheidung zwischen den vom Probenanalyten und den vom Indikatoranalyten hervorgerufenen Signalen in der Regel nicht möglich ist. Somit erweist es sich als vorteilhaft, wenn der Indikatoranalyt alternativ erst nach vollständiger Umsetzung des Probenanalyten durch das Nachweisreagenz freigesetzt und das erzeugte Signal im Rahmen eines Total-System-Checks gemessen wird.

Vorzugsweise werden im Rahmen der vorliegenden Erfindung diagnostische Elemente eingesetzt, auf den der Analyt in Form einer wässrigen oder nichtwässrigen Lösung aufgebracht werden kann. In einer besonders bevorzugten Ausführungsform der Erfindung handelt es sich bei dem diagnostischen Element um ein Testband, eine Testdisc, ein Testpad, einen Teststreifen, eine Teststreifentrommel, oder um die in WO 2005/084530 A2 genannten diagnostischen Elemente, auf welche hiermit ausdrücklich Bezug genommen wird. Die in vorliegender Anmeldung beschriebenen diagnostischen Elemente umfassen hierbei jeweils mindestens einen Testbereich, welcher mit einer den Analyten enthaltenden Probe in Kontakt gebracht werden kann und unter Verwendung geeigneter Mittel eine qualitative oder/und quantitative Bestimmung des Analyten ermöglicht.

Der Begriff "Testband", wie er hierin verwendet wird, bezeichnet ein bandförmiges diagnostisches Element, welches üblicherweise mehr als einen einzelnen Testbereich, bevorzugt mindestens 10 einzelne Testbereiche, stärker bevorzugt mindestens 25 einzelne Testbereiche, und am stärksten bevorzugt mindestens 50 einzelne Testbereiche umfasst. Vorzugsweise sind die einzelnen Testbereiche jeweils in einem Abstand von wenigen Millimetern bis zu wenigen Zentimetern, beispielsweise in einem Abstand von < 2.5 cm, voneinander angeordnet, wobei das Testband zwischen aufeinander folgenden Testbereichen gegebenenfalls Markierungsbereiche zur Wegerfassung beim Bandtransport oder/und zur Kalibrierung umfassen kann. Derartige Testbänder sind beispielsweise in EP 1 739 432 A1 beschrieben, auf deren Offenbarung ausdrücklich Bezug genommen wird.

Der Begriff "Testdisc", wie er hierin verwendet wird, bezeichnet ein scheibenförmiges diagnostisches Element, welches einen oder mehrere einzelne Testbereiche, beispielsweise mindestens 10 einzelne Testbereiche, umfassen kann. In einer Ausführungsform ist die Testdisc mit einer dünnen Schicht der Testchemie, beispielsweise mit einer Schicht in einer Dicke von etwa 20 µm, überzogen, auf welche eine Probe des Analyten aufgebracht werden kann, wobei in Abhängigkeit vom Volumen der Probe ein mehr oder weniger großer Bereich der Testdisc von der Probe benetzt wird und zur Bestimmung des Analyten verwendet werden kann. Der nicht-benetzte Bereich der Testdisc, welcher infolge des Durchtritts von Feuchtigkeit durch die Testchemieschicht teilweise oder vollständig befeuchtet sein kann, steht in der Folge für weitere Bestimmungen des Analyten zur Verfügung.

Die erfindungsgemäßen Testelemente können zur Bestimmung einer beliebigen biologischen oder chemischen Substanz verwendet werden, welche photochemisch oder elektrochemisch nachweisbar ist. Vorzugsweise wird der Analyt aus der Gruppe bestehend aus Äpfelsäure, Alkohol, Ammonium, Ascorbinsäure, Cholesterin, Cystein, Glucose, Glutathion, Glycerin, Harnstoff, 3-Hydroxybutyrat, Milchsäure, 5'-Nukleotidase, Peptiden, Pyruvat, Salicylat und Triglyceriden ausgewählt, wobei Glucose besonders bevorzugt ist. Der Analyt kann dabei aus einer beliebigen Quelle stammen, ist jedoch bevorzugt in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, enthalten. Bevorzugt wird mittels der hierin beschriebenen diagnostischen Elemente das Vorhandensein und/oder die Menge eines Analyten in einer Probe aus Vollblut, Plasma, Serum oder extrazellulärer Gewebeflüssigkeit bestimmt.

In einer bevorzugten Ausführungsform sieht die Erfindung vor, dass die hierin beschriebenen diagnostischen Elemente zur Bestimmung mehrerer Analyten ausgebildet sind, wobei der Begriff "mehrere", wie er hierin verwendet wird, jegliche Zahl > 1, bevorzugt 2 bis 10, stärker bevorzugt 2 bis 5, am stärksten bevorzugt 2 oder 3, bedeutet. Sofern mehrere Analyten, welche in einer einzigen oder mehreren unterschiedlichen Proben enthalten sein können, mittels der erfindungsgemäßen Testelemente bestimmt werden sollen, so kann die Bestimmung der unterschiedlichen Analyten grundsätzlich in ein und demselben Testbereich oder in unterschiedlichen Testbereichen eines diagnostischen Elements erfolgen, wobei eine Bestimmung von mehreren Analyten in einem einzigen Testbereich als vorteilhaft angesehen wird. Zu diesem Zweck können die erfindungsgemäßen Testelemente beispielsweise für jeden zu bestimmenden Analyten ein hierfür spezifisches Nachweisreagenz sowie eine definierte Menge des jeweiligen Analyten umfassen und insbesondere zur aufeinander folgenden Bestimmung der einzelnen Analyten, beispielsweise durch Freisetzen der jeweiligen Indikatoranalyten in dem diagnostischen Element aus unterschiedlichen Schichten oder/und zu unterschiedlichen Zeitpunkten, ausgebildet sein.

Die qualitative oder/und quantitative Bestimmung des Analyten kann auf beliebige Art und Weise erfolgen. Hierzu können grundsätzlich alle aus dem Stand der Technik bekannten Methoden zum Nachweis enzymatischer Reaktionen eingesetzt werden, welche ein messbares Signal erzeugen, das manuell oder unter Verwendung geeigneter Mittel ausgewertet bzw. ausgelesen werden kann. Im Rahmen der vorliegenden Erfindung gelangen bevorzugt optische Nachweismethoden, welche beispielsweise die Messung von Absorption, Fluoreszenz, Circulardichroismus (CD), optische Rotationsdispersion (ORD), Refraktometrie, etc. umfassen, sowie elektrochemische Techniken zur Anwendung. Besonders bevorzugt erfolgt der Nachweis des Analyten photometrisch oder fluorometrisch, beispielsweise indirekt über eine fluorometrisch detektierbare Veränderung des Coenzyms.

In einem weiteren Aspekt betrifft die Erfindung ein analytisches Messgerät, welches ein erfindungsgemäßes diagnostisches Element umfasst und zur qualitativen oder/und quantitativen Bestimmung eines Analyten dient. Beispiele für derartige analytische Messgeräte umfassen u.a. die kommerziell erhältlichen Produkte Accu-Chek^{®} Active, Accu-Chek^{®} Compact und Accu-Chek^{®} Mobile (alle Fa. Roche), sind jedoch nicht auf diese beschränkt.

In noch einem weiteren Aspekt betrifft die Erfindung ein Verfahren zur Bestimmung eines Analyten, umfassend die Schritte:
(a) Inkontaktbringen des Analyten mit einem erfindungsgemäßen diagnostischen Element, und
(b) Bestimmen des Vorhandenseins oder/und der Menge des Analyten.

In noch einem weiteren Aspekt betrifft die Erfindung ein Verfahren zur Berichtigung eines von einem Analyten erzeugten Signals, umfassend die Schritte:
(a) Einbringen eines erfindungsgemäßen diagnostischen Elements in ein analytisches Messgerät,
(b) Erzeugen eines ersten detektierbaren Signals in dem analytischen Messgerät durch Inkontaktbringen des Analyten mit dem diagnostischen Element,
(c) Erzeugen eines zweiten detektierbaren Signals in dem analytischen Messgerät durch Freisetzen des Indikatoranalyten in dem diagnostischen Element, und
(d) Berichtigen des in Schritt (b) erzeugten Signals unter Verwendung des in Schritt (c) erzeugten Signals.

Im Rahmen des oben beschriebenen Verfahrens wird zunächst ein erfindungsgemäßes diagnostisches Element in ein analytisches Messgerät, wie es beispielsweise vorstehend beschrieben ist, eingebracht. Anschließend wird der zu bestimmende Analyt mit dem diagnostischen Element in Kontakt gebracht, wobei der Analyt und das für den Analyten spezifische Nachweisreagenz miteinander reagieren und ein erstes, vorzugsweise optisch oder elektrochemisch detektierbares Signal in dem analytischen Messgerät erzeugt wird.

Um zu evaluieren, inwieweit das erste Messsignal die Konzentration des Analyten in der Probe korrekt darstellt, wird in einem weiteren Schritt der Indikatoranalyt in dem diagnostischen Element aus seiner für eine Reaktion mit dem Nachweisreagenz unzugänglichen Form freigesetzt, wobei die Freisetzung vorzugsweise mittels Methoden erfolgt, wie sie in Zusammenhang mit der Beschreibung der erfindungsgemäßen diagnostischen Elemente genannt sind. Der auf diese Weise freigesetzte Indikatoranalyt kann sodann mit dem für den Analyten spezifischen Nachweisreagenz in Kontakt treten, wobei in dem analytischen Messgerät ein zweites, vorzugsweise optisch oder elektrochemisch detektierbares Signal erzeugt wird, welches aufgrund der gleichzeitigen Detektion von Analyt und freigesetztem Indikatoranalyt üblicherweise stärker ausgeprägt ist als das erste Messsignal.

Unter Verwendung geeigneter Mittel, wie beispielsweise einer Kalibrationskurve, können das erste und das zweite Messsignal, welche vorzugsweise mittels zweier unterschiedlicher Messkanäle eines geeigneten analytischen Messgeräts detektiert werden, jeweils mit einer bestimmten Konzentration des Analyten in Beziehung gesetzt werden. Sofern die aus dem ersten Messsignal berechnete Konzentration des Analyten mit der aus dem zweiten Messsignal berechneten Konzentration des Analyten nicht übereinstimmt, so wird die aus dem ersten Messsignal berechnete Konzentration des Analyten unter Verwendung des Ergebnisses der zweiten Messung berichtigt, was beispielsweise mittels eines geeigneten Algorithmus bewerkstelligt werden kann. Auf diese Weise besteht die Möglichkeit, potenzielle Schwankungen der Messwerte, welche u.a. durch umweltbedingte Einflüsse (z. B. Temperatur, Luftfeuchtigkeit), durch herstellungsbedingte Unregelmäßigkeiten der diagnostischen Elemente oder/und durch die Anwesenheit von interferierenden Substanzen in der Probe bedingt sein können, zu minimieren und damit die Genauigkeit der Analytbestimmung zu verbessern.

In noch einem weiteren Aspekt betrifft die Erfindung ein Verfahren zur Überprüfung der Detektionsoptik eines analytischen Messgeräts, umfassend die Schritte:
(a) Einbringen eines erfindungsgemäßen diagnostischen Elements in das analytische Messgerät,
(b) Erzeugen eines detektierbaren Signals in dem analytischen Messgerät durch Freisetzen des Indikatoranalyten in dem diagnostischen Element, und
(c) Korrelieren des in Schritt (b) erzeugten Signals mit einem Referenzsignal.

Das Verfahren dient insbesondere der Überprüfung einer Verschmutzung der Detektionsoptik eines analytischen Messgeräts. Nach Einbringen eines erfindungsgemäßen diagnostischen Elements in das analytische Messgerät und Freisetzen des Indikatoranalyten in dem diagnostischen Element beispielsweise mittels Methoden, wie sie in Zusammenhang mit der Beschreibung der erfindungsgemäßen diagnostischen Elemente genannt sind, wird in dem analytischen Messgerät durch Reaktion des freigesetzten Indikatoranalyten mit dem für den Analyten spezifischen Nachweisreagenz ein definiertes, vorzugsweise optisch oder elektrochemisch detektierbares Signal erzeugt, das mit einem Referenzsignal in Korrelation gesetzt werden kann.

Sofern die Detektionsoptik des analytischen Messgeräts verschmutzt ist, wird bei Umsetzung des Indikatoranalyten mit dem Nachweisreagenz ein gegenüber dem Referenzsignal schwächeres Signal detektiert, in dessen Folge der Anwender, beispielsweise durch eine optische oder akustische Meldung, auf eine Beeinträchtigung des analytischen Messgeräts aufmerksam gemacht und der Messwert alsdann verworfen werden kann.

In noch einem weiteren Aspekt betrifft die Erfindung ein System zur kontrollierten Freisetzung eines Reagenzes, umfassend
(a) einen Träger, umfassend das Reagenz und ein davon separates Reaktionssystem, wobei das Reagenz in einer für eine Reaktion mit dem Reaktionssystem unzugänglichen, freisetzbaren Form vorliegt und unter definierten Bedingungen freisetzbar ist, und
(b) Mittel zur Freisetzung des Reagenzes auf dem Träger.

In noch einem weiteren Aspekt betrifft die Erfindung die Verwendung eines erfindungsgemäßen Freisetzungssystems als Schaltelement.

Die Erfindung soll durch die nachfolgenden Figuren und Beispiele näher erläutert werden:

### Beschreibung der Figuren

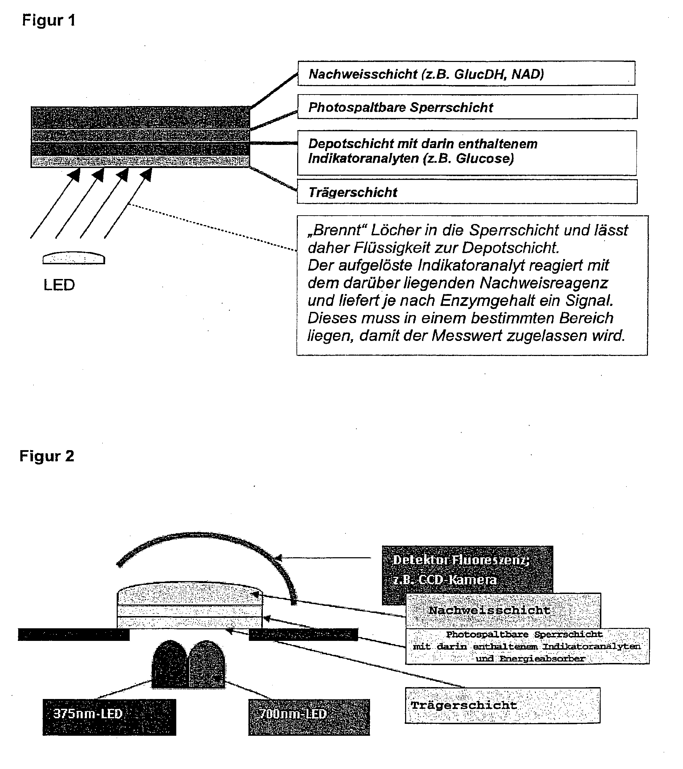

- **Figur 1:**: Querschnitt einer Ausführungsform eines diagnostischen Elements gemäß der vorliegenden Erfindung, umfassend eine Nachweisschicht, eine photochemisch spaltbare Sperrschicht, eine Depotschicht mit darin enthaltenem Indikatoranalyten, und eine Trägerschicht.
- **Figur 2:**: Querschnitt einer alternativen Ausführungsform eines diagnostischen Elements gemäß der vorliegenden Erfindung, umfassend eine Nachweisschicht, eine photochemisch spaltbare Sperrschicht mit darin enthaltenem Indikatoranalyten und Energieabsorber, und eine Trägerschicht.

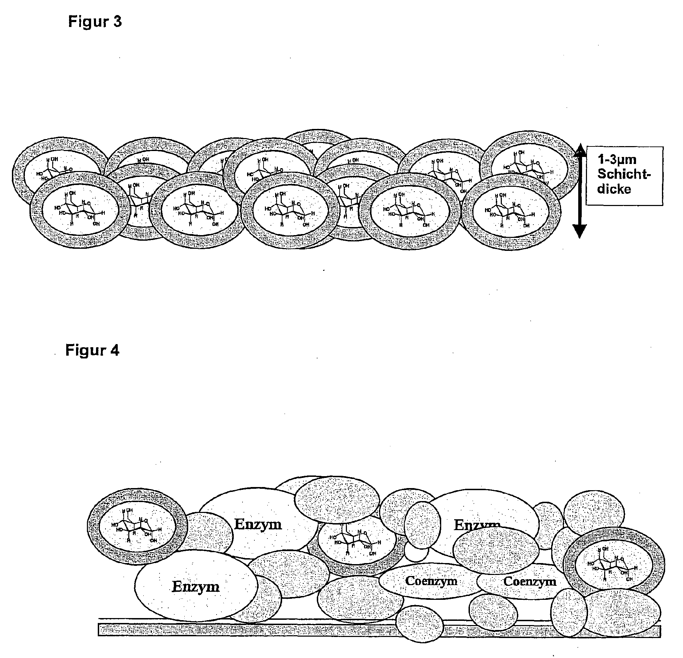

- **Figur 3:**: Querschnitt der Schutzschicht eines diagnostischen Elements gemäß der vorliegenden Erfindung, welche den Indikatoranalyten (hier Glucose) in verkapselter Form enthält.
- **Figur 4:**: Querschnitt der Nachweisschicht eines diagnostischen Elements gemäß der vorliegenden Erfindung, welche den Indikatoranalyten (hier Glucose) in verkapselter Form enthält.

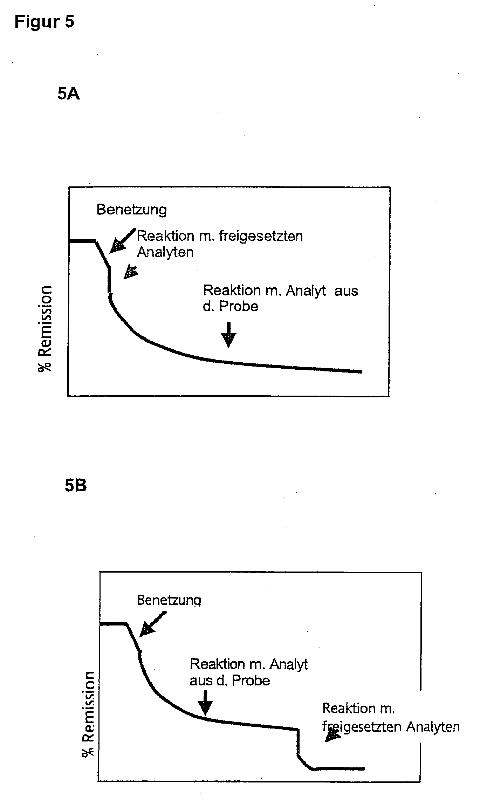

- **Figur 5:**: Kinetik der Umsetzung von Indikatoranalyt bzw. Probenanalyt durch das Enzymsystem eines diagnostischen Elements gemäß der vorliegenden Erfindung.
**5A:** Freisetzung des Indikatoranalyten vor Beginn der Umsetzung des Probenanalyten durch das Enzymsystem.
**5B:** Freisetzung des Indikatoranalyten nach Beendigung der Umsetzung des Probenanalyten durch das Enzymsystem.
- **Figur 6:**: Fluorometrischer Nachweis von Indikatoranalyt durch photochemische Freisetzung und anschließende enzymatische Umsetzung.
**6A:** Fluoreszenz eines den Indikatoranalyten in derivatisierter Form enthaltenden, mit Licht geeigneter Wellenlänge bestrahlten diagnostischen Elements vor bzw. nach Inkontaktbringen mit einer Lösung von Enzym (GlucDH) und Coenzym (NAD).
**6B:** Fluoreszenz eines den Indikatoranalyten nicht enthaltenden diagnostischen Elements in Abwesenheit bzw. Gegenwart einer Lösung von Enzym (GlucDH) und Coenzym (NAD).

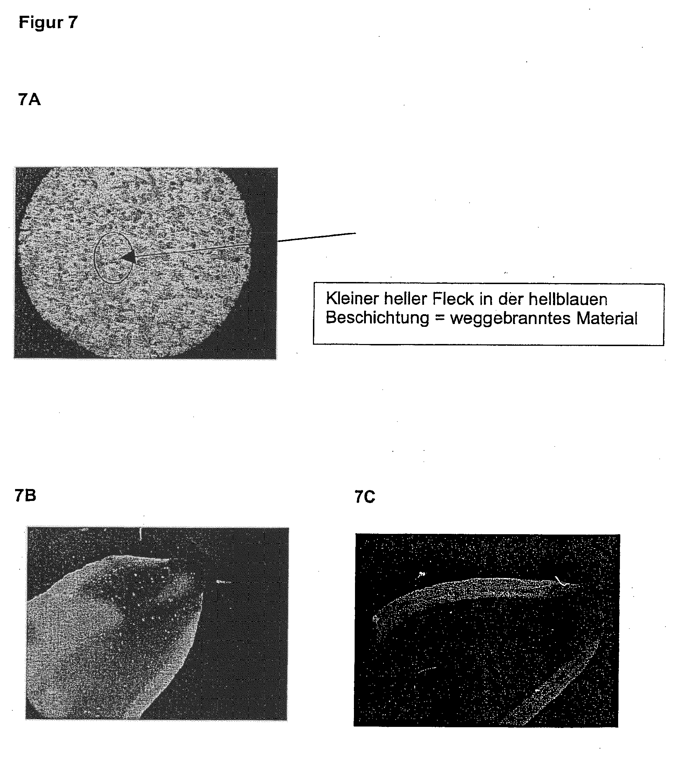

- **Figur 7:**: Fluorometrischer Nachweis von Indikatoranalyt durch thermische Lochbildung und anschließende enzymatische Umsetzung.
**7A:** .Digitalfotoaufnahme einer mit Farbstoff (Cryptocyanin) dotierten Polymerschicht nach 20-minütigem Bestrahlen mit Licht geeigneter Wellenlänge.
**7B:** Digitalfotoaufnahme einer mit Farbstoff (Cryptocyanin) dotierten Polymerschicht nach 20-minütigem Bestrahlen mit Licht geeigneter Wellenlänge und anschließendem Inkontaktbringen mit einer Lösung von Enzym (GlucDH) und Coenzym (NAD).
**7C:** Digitalfotoaufnahme einer mit Farbstoff (Cryptocyanin) dotierten Polymerschicht nach Inkontaktbringen mit einer Lösung von Enzym (GlucDH) und Coenzym (NAD) ohne vorheriges Bestrahlen.

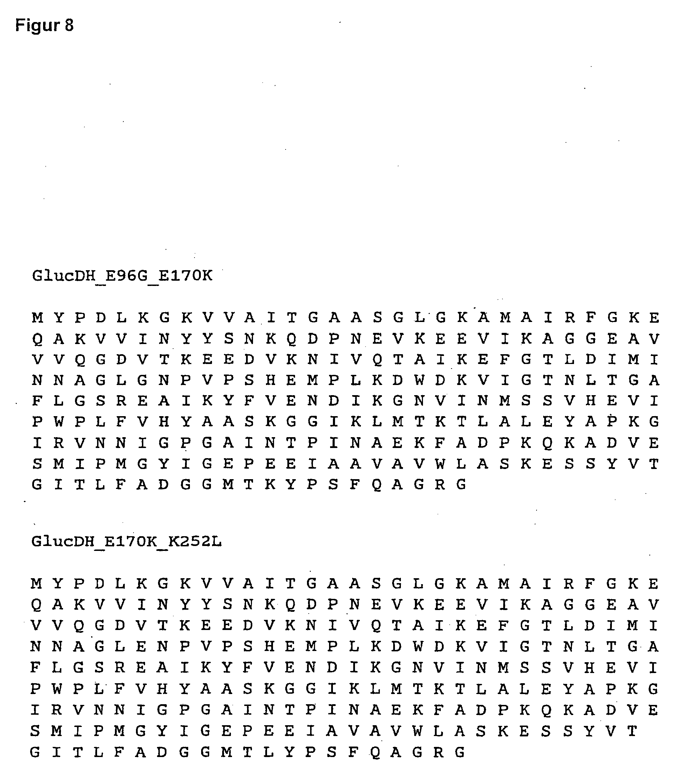

- **Figur 8:**: Darstellung der Aminosäuresequenzen der Glucose-Dehydrogenase-Doppelmutanten GlucDH_E96G_E170K und GlucDH_E170K_K252L.

### Beispiele

### Beispiel 1: Herstellung und Anwendung eines diagnostischen Elements mit Indikatoranalyt in derivatisierter Form

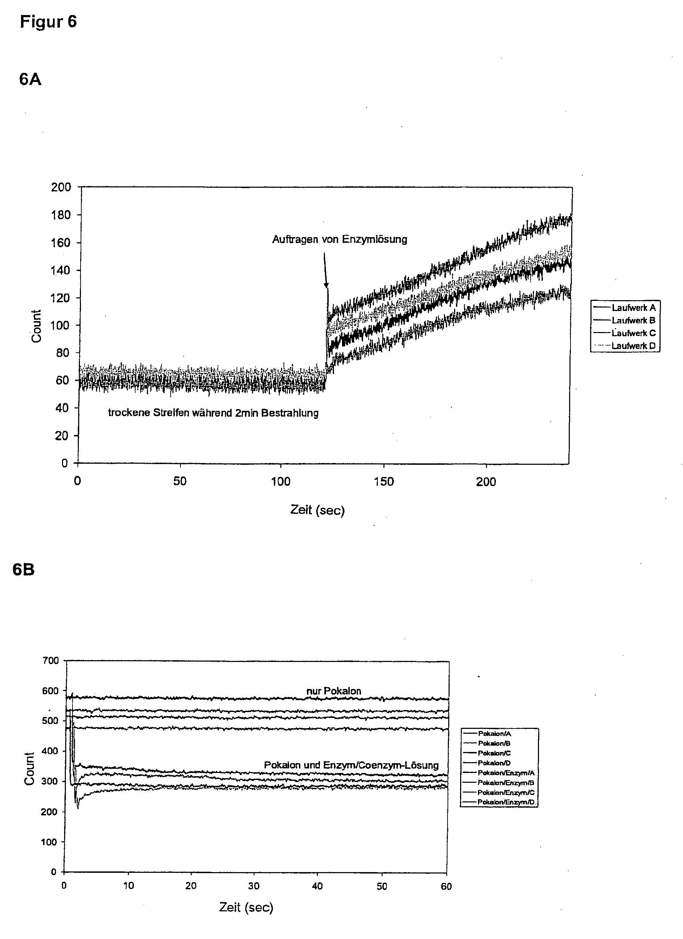

Glucose in derivatisierter Form (Tris-Veratryl-Derivat) und Polyacrylamid (Firma Aldrich) wurden zusammen auf eine Trägerschicht aus Pokalonfolie (Firma Lonza) aufgebracht. Anschließend wurde das auf diese Weise erhaltene, zweischichtige Testelement auf ein Lumineszenzmessgerät (Eigenbau Firma Roche) platziert und zwei Minuten mit Licht einer Wellenlänge von 375 nm bestrahlt. Zum Nachweis der Freisetzung von Glucose aus dem im Testelement eingesetzten Glucosederivat wurde alsdann eine Lösung von 10 mg Glucose-Dehydrogenase (GlucDH, Firma Roche) und 10 mg Nikotinamid-Adenin-Dinukleotid (NAD; Firma Roche) in 10 ml Phosphatpuffer, pH 7 (Firma Merck) auf das diagnostische Element aufgebracht und die Bildung von NADH fluorometrisch verfolgt (siehe Figur 6A).

Als Referenz wurden die leere Pokalonfolie bzw. die Pokalonfolie in Kombination mit der oben beschriebenen Lösung von GlucDH und NAD jeweils fluorometrisch vermessen (siehe Figur 6B).

### Beispiel 2: Herstellung und Anwendung eines diagnostischen Elements mit freiem Indikatoranalyten und farbstoffdotierter Polymermatrix

Zur Bereitstellung eines diagnostischen Elements mit farbstoffdotierter Polymermatrix wurden zwei Teillösungen 1 und 2 hergestellt, welche die nachfolgenden Zusammensetzungen aufwiesen:
Teillösung 1:

| | Einwaage (g) | Feststoffgehalt (%) | Feststoff (g) | % auf GFS |
|---|---|---|---|---|
| Teillösung 1 | 100,00 | | | |
| PAA 1500 (Polyacrylamid) | 60,00 | 50 | 30,00 | 33,33 |
| Glucose | 40,00 | 100 | 40,00 | 44,44 |

Teillösung 2:

| | Einwaage (g) | Feststoffgehalt (%) | Feststoff (g) | % auf GFS |
|---|---|---|---|---|
| Teillösung 2 | 4,41 | | | |
| 10%ige Lösung von PEMA in Chloroform | 4,00 | 10 | 0,4 | 49,57 |
| Cryptocyanin | 0,11 | 100 | 0,107 | 13,26 |
| Azo-bis(cyclohexyl)carbodiimid | 0,30 | 100 | 0,30 | 37,17 |

Nach Bereistellung der beiden Teillösungen wurde aus Teillösung 1 durch Aufrakeln (30 µm Nassschichtdicke) und anschließendes Trocknen eine glucosehaltige Schicht erzeugt. Auf die getrocknete, den Indikatoranalyten enthaltende Schicht wurde im Anschluss Teillösung 2 aufgerakelt (60 µm Nassschichtdicke) und getrocknet.

Die auf diese Weise erhaltene farbstoffdotierte Polymerschicht wurde mit einem Handlaser (Wellenlänge 650 nm, < 5 Watt) bestrahlt, wobei nach ca. 20 Minuten Löcher in der bestrahlten Schicht beobachtet werden konnten (siehe Figur 7A). Anschließend wurde auf die durch Bestrahlung erzeugten Löcher eine Lösung von 10 mg Glucose-Dehydrogenase (GlucDH, Firma Roche) und 10 mg Nikotinamid-Adenin-Dinukleotid (NAD, Firma Roche) in 10 ml Phosphatpuffer, pH 7 (Firma Merck) aufgebracht, wobei die aus der unteren Schicht freigesetzte Glucose durch Reaktion mit dem Enzymsystem oxidiert und grünlich-blau fluoreszierendes NADH gebildet wurde (siehe Figur 7B).

Als Referenz wurde obige Lösung von Glucose-Dehydrogenase (GlucDH) und Nikotinamid-Adenin-Dinukleotid (NAD) in Phosphatpuffer, pH 7 ohne vorheriges Bestrahlen der farbstoffdotierten Polymerschicht auf letztere Schicht aufgebracht. Hierbei zeigte sich, dass mangels thermisch induzierter Lochbildung keine Glucose aus der unteren Schicht freigesetzt und kein NADH gebildet wird (siehe Figur 7C).

## Patentansprüche

1. Diagnostisches Element zur Bestimmung mindestens eines Analyten, umfassend
(a) ein für den Analyten spezifisches Nachweisreagenz, und
(b) eine definierte Menge des zu bestimmenden Analyten (Indikatoranalyt), wobei der Indikatoranalyt in einer für eine Reaktion mit dem Nachweisreagenz unzugänglichen, freisetzbaren Form vorliegt.

2. Diagnostisches Element nach Anspruch 1,
**dadurch gekennzeichnet,**
**dass** das Nachweisreagenz ein Enzym oder/und ein Coenzym umfasst, wobei das Enzym vorzugsweise eine Nikotinamid-Adenin-Dinukleotid (NAD/NADH)- oder Nikotinamid-Adenin-Dinukleotidphosphat (NADP/NADPH)-abhängige Dehydrogenase, insbesondere eine Glucose-Dehydrogenase (EC 1.1.1.47) oder eine Glucose-6-phosphat-Dehydrogenase (EC 1.1.1.49), ist,
oder/und das Coenzym vorzugsweise eine stabilisierte Nikotinamid-Adenin-Dinukleotid (NAD/NADH)- oder Nikotinamid-Adenin-Dinukleotidphosphat (NADP/NADPH)-Verbindung, insbesondere carbaNAD oder carbaNADP, oder die Verbindung der Formel (I) 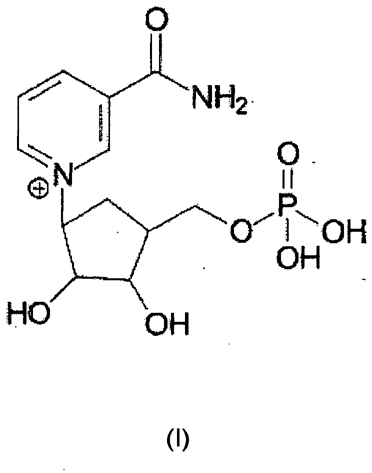 ist.

3. Diagnostisches Element nach Anspruch 1 oder 2,
**dadurch gekennzeichnet,**
**dass** das Nachweisreagenz weiterhin einen Mediator oder/und einen optischen Indikator umfasst, oder/und der Indikatoranalyt eine photochemisch oder/und elektrochemisch spaltbare Schutzgruppe umfasst.

4. Diagnostisches Element nach einem der Ansprüche 1 bis 3,
**dadurch gekennzeichnet,**
**dass** es eine Trägerschicht sowie eine das Nachweisreagenz enthaltende Nachweisschicht umfasst, oder/und zur Bestimmung mehrerer Analyten ausgebildet ist.

5. Diagnostisches Element nach Anspruch 4,
**dadurch gekennzeichnet,**
**dass** es den Indikatoranalyten in der Nachweisschicht oder in einer davon separaten Depotschicht enthält, wobei vorzugsweise eine auflösbare Sperrschicht zwischen der Depotschicht und der Nachweisschicht angeordnet ist.

6. Diagnostisches Element nach Anspruch 4 oder 5,
**dadurch gekennzeichnet,**
**dass** die Nachweisschicht, die Sperrschicht oder/und die Depotschicht eine Polymermatrix umfasst, welche mindestens einen Energieabsorber enthält, wobei der Energieabsorber vorzugsweise ein hydrophober Farbstoff ausgewählt aus einem Cyaninfarbstoff, insbesondere Cryptocyanin, Indotricarbocyanin (C7), Oxacarbocyanin (C3), Pinacyanoliodid, Thiacarbocyanin (C3), Thiadicarbocyanin (C5), und einem Squarylium-Farbstoff, insbesondere Squarylium-Farbstoff III, ist.

7. Diagnostisches Element nach Anspruch 6,
**dadurch gekennzeichnet,**
**dass** die Polymermatrix aus mindestens einem hydrophoben Polymer, insbesondere einem hydrophoben Polymer ausgewählt aus der Gruppe bestehend aus einem Polymethylmethacrylat, einem Polyethylmethacrylat, einem Polycarbonat und chemischen Derivaten hiervon, gebildet ist, oder/und den mindestens einen Energieabsorber kovalent gebunden oder/und in freier Form enthält.

8. Diagnostisches Element nach Anspruch 6 oder 7,
**dadurch gekennzeichnet,**
**dass** der Indikatoranalyt in der Polymermatrix verkapselt ist.

9. Diagnostisches Element nach einem der Ansprüche 1 bis 8,
**dadurch gekennzeichnet,**
**dass** es als Testband, Testdisc, Testpad, Teststreifen oder Teststreifentrommel ausgebildet ist.

10. Analytisches Messgerät, umfassend ein diagnostisches Element nach einem der Ansprüche 1 bis 9.

11. Verfahren zur Bestimmung eines Analyten, umfassend die Schritte:
(a) Inkontaktbringen des Analyten mit einem diagnostischen Element nach einem der Ansprüche 1 bis 9, und
(b) Bestimmen des Vorhandenseins oder/und der Menge des Analyten.

12. Verfahren zur Berichtigung eines von einem Analyten erzeugten Signals, umfassend die Schritte:
(a) Einbringen eines diagnostischen Elements nach einem der Ansprüche 1 bis 9 in ein analytisches Messgerät,
(b) Erzeugen eines ersten detektierbaren Signals in dem analytischen Messgerät durch Inkontaktbringen des Analyten mit dem diagnostischen Element,
(c) Erzeugen eines zweiten detektierbaren Signals in dem analytischen Messgerät durch Freisetzen des Indikatoranalyten in dem diagnostischen Element, und
(d) Berichtigen des in Schritt (b) erzeugten Signals unter Verwendung des in Schritt (c) erzeugten Signals.

13. Verfahren zur Überprüfung der Detektionsoptik eines analytischen Messgeräts, umfassend die Schritte:
(a) Einbringen eines diagnostischen Elements nach einem der Ansprüche 1 bis 9 in das analytische Messgerät,
(b) Erzeugen eines detektierbaren Signals in dem analytischen Messgerät durch Freisetzen des Indikatoranalyten in dem diagnostischen Element, und
(c) Korrelieren des in Schritt (b) erzeugten Signals mit einem Referenzsignal.

14. System zur kontrollierten Freisetzung eines Reagenzes für den spezifischen Nachweis eines Analyten, umfassend
(a) einen Träger, umfassend das Reagenz und ein davon separates Reaktionssystem, wobei das Reagenz in einer für eine Reaktion mit dem Reaktionssystem unzugänglichen, freisetzbaren Form vorliegt und unter definierten Bedingungen freisetzbar ist,
(b) Mittel zur Freisetzung des Reagenzes auf dem Träger.

15. System nach Anspruch 14,
**dadurch gekennzeichnet,**
**dass** das Reagenz in einer auflösbaren Matrix verkapselt ist, durch eine auflösbare Sperrschicht von dem Reaktionssystem abgetrennt ist, oder/und eine chemische Schutzgruppe umfasst.

16. Verwendung eines Systems nach Anspruch 14 oder 15 als Schaltelement.

## Claims

1. Diagnostic element for determining at least one analyte, comprising
(a) a specific detection reagent for the analyte and
(b) a defined amount of the analyte to be determined (indicator analyte), in which the indicator analyte is present in a form so that it may be released and is inaccessible for a reaction with the detection reagent.

2. Diagnostic element according to claim 1,
**characterised in that**
the detection reagent comprises an enzyme and/or a coenzyme, in which the enzyme is preferably a nicotinamide-adenine-dinucleotide (NAD/NADH) or nicotinamide-adenine-dinucleotide phosphate (NADP/NADPH) dependent dehydrogenase, particularly a glucose dehydrogenase (EC 1.1.1.47) or a glucose-6-phosphate dehydrogenase (EC 1.1.1.49),
and/or the coenzyme is preferably a stabilised nicotinamide-adenine-dinucleotide (NAD/NADH) or nicotinamide-adenine-dinucleotide phosphate (NADP/NADPH) compound, particularly carbaNAD or carbaNADP, or the compound according to formula (I).

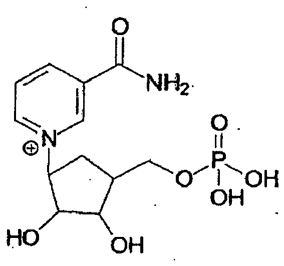

3. Diagnostic element according to claim 1 or 2,
**characterised in that**
the detection reagent also comprises a mediator and/or an optical indicator and/or the indicator analyte comprises a protection group that may be split photochemically and/or electrochemically.

4. Diagnostic element according to one of claims 1 to 3,
**characterised in that**
it comprises a carrier layer as well as a detection layer containing the detection reagent and/or is made for determining several analytes.

5. Diagnostic element according to claim 4,
**characterised in that**
it contains the indicator analytes in the detection layer or in a deposit layer that is separate from it, in which preferably a barrier layer that may be dissolved is arranged between the deposit layer and the detection layer.

6. Diagnostic element according to claim 4 or 5,
**characterised in that**
the detection layer, the barrier layer or/and the deposit layer comprises a polymer matrix, which contains at least one energy absorber, in which the energy absorber is preferably a hydrophobic dye selected from a cyanine dye, particularly cryptocyanine, indotricarbocyanine (C7), oxacarbocyanine (C3), pinacyanol iodide, thiacarbocyanine (C3), thiadicarbocyanine (C5), and a squarylium dye, particularly squarylium dye III.

7. Diagnostic element according to claim 6,
**characterised in that**
the polymer matrix is made of at least one hydrophobic polymer, particularly a hydrophobic polymer selected from the group consisting of a polymethyl methacrylate, a polyethyl methacrylate, a polycarbonate and chemical derivatives of them and/or contains the at least one energy absorber bonded covalently and/or in free form.

8. Diagnostic element according to claim 6 or 7,
**characterised in that**
the indicator analyte is encapsulated in the polymer matrix.

9. Diagnostic element according to one of claims 1 to 8,
**characterised in that**
it is made as a test band, test disc, test path, test strip or test strip drum.

10. Analytical measuring device, comprising a diagnostic element according to one of claims 1 to 9.

11. Method for determining an analyte, comprising the steps:
(a) Bringing the analyte into contact with a diagnostic element according to one of claims 1 to 9, and
(b) Determining the presence and/or the amount of the analyte.

12. Method for correcting a signal produced by an analyte, comprising the steps:
(a) Introducing a diagnostic element according to one of claims 1 to 9 into an analytical measuring device,
(b) Producing a first signal that may be detected in the analytical measuring device by bringing the analyte into contact with the diagnostic element,
(c) Producing a second signal that may be detected in the analytical measuring device by releasing the indicator analyte in the diagnostic element and
(d) Correcting the signal produced in step (b) using the signal produced in step (c).

13. Method for examining the detection optics of an analytical measuring device, comprising the steps:
(a) Introducing a diagnostic element according to one of claims 1 to 9 into the analytical measuring device,
(b) Producing a signal that may be detected in the analytical measuring device by releasing the indicator analyte in the diagnostic element and
(c) Correlating the signal produced in step (b) with a reference signal.

14. System for controlled release of a reagent for the specific detection of an analyte, comprising
(a) a carrier, comprising the reagent and a reaction system that is separate from it, in which the reagent is present in a form so that it may be released and is inaccessible for a reaction with the reaction system and may be released under defined conditions,
(b) means for releasing the reagent on the carrier.

15. System according to claim 14,
**characterised in that**
the reagent is encapsulated in a matrix that may be dissolved, is separated from the reaction system by a barrier layer that may be dissolved and/or comprises a chemical protection group.

16. Use of a system according to claim 14 or 15 as a switching element.

## Revendications

1. Elément de diagnostic pour la détermination d'au moins un analyte, comprenant
(a) un réactif de détection spécifique à l'analyte, et
(b) une quantité définie de l'analyte à déterminer (analyte indicateur), dans laquelle l'analyte indicateur est présent sous une forme libérable inaccessible pour une réaction avec le réactif de détection.

2. Elément de diagnostic selon la revendication 1,
**caractérisé en ce que**
le réactif de détection comprend une enzyme et/ou une co-enzyme, dans lequel l'enzyme est de préférence une déshydrogénase dépendante de nicotinamide-adénine-dinucléotide (NAD / NADH) ou de nicotinamide-adénine-dinucléotide phosphate (NADP / NADPH), en particulier une glucose déshydrogénase (EC 1.1.1.47), ou une glucose-6 phosphate déshydrogénase (EC 1.1.1.49),
ou/et dans lequel la co-enzyme est de préférence un composé stabilisé de nicotinamide-adénine-dinucléotide (NAD / NADH) ou de nicotinamide-adénine-dinucléotide phosphate (NADP / NADPH), en particulier carbaNAD ou carbaNADP, ou un composé de formule (I)

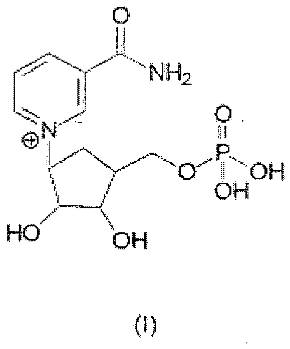

3. Elément de diagnostic selon la revendication 1 ou 2,
**caractérisé en ce que**
le réactif de détection comprend de plus un médiateur ou/et un indicateur optique, ou/et **en ce que** l'analyte indicateur comprend un groupement protecteur photochimiquement et / ou électro-chimiquement clivable.

4. Elément de diagnostic selon l'une quelconque des revendications 1 à 3,
**caractérisé en ce**
**qu'**il comprend une couche support ainsi qu'une couche de détection contenant le réactif de détection, ou/et en ce qu'il est prévu pour la détermination de plusieurs analytes.

5. Elément de diagnostic selon la revendication 4,
**caractérisé en ce**
**qu'**il contient l'analyte indicateur dans la couche de détection ou dans un couche de dépôt séparée de celle-ci, dans lequel de préférence une couche barrière soluble entre la couche de dépôt et la couche de détection est prévue.

6. Elément de diagnostic selon la revendication 4 ou 5,
**caractérisé en ce**
**que** la couche de détection, la couche barrière ou/et la couche de dépôt comprend une matrice de polymères, contenant au moins un absorbeur d'énergie, dans lequel l'absorbeur d'énergie de préférence est un colorant hydrophobe choisi parmi un colorant cyanine, en particulier la cryptocyanine, l'indotricarbocyanine (C7), l'oxacarbocyanine (C3), l'iodure de pinacyanol, la thiacarbocyanine (C3), la thiadicarbocyanin (C5), et un colorant de squarylium, en particulier le colorant de squarylium III.

7. Elément de diagnostic selon la revendication 6,
**caractérisé en ce**
**que** la matrice de polymères est formée à partir d'un polymère hydrophobe, en particulier un polymère hydrophobe choisi parmi le groupe constitué par un polyméthacrylate de méthyle, un polyméthacrylate d'éthyle, un polycarbonate et des dérivés chimiques de ceux-ci, ou/et elle contient au moins un absorbeur d'énergie lié de façon covalente ou/et sous forme libre.

8. Elément de diagnostic selon la revendication 6 ou 7,
**caractérisé en ce**
**que** l'analyte indicateur est encapsulé dans la matrice de polymères.

9. Elément de diagnostic selon l'une quelconque des revendications 1 à 8,
**caractérisé en ce**
**qu'**il est conçu comme une bande de test, un disque de test, un tampon de test, une bandelette de test ou une cartouche de bandelettes de test.

10. Instrument d'analyse, comprenant un élément de diagnostic selon l'une quelconque des revendications 1 à 9.

11. Procédé pour la détermination d'un analyte, comprenant les étapes consistant à:
(a) mettre en contact l'analyte avec un élément de diagnostic selon l'une des revendications 1 à 9, et
(b) déterminer la présence ou/et la quantité de l'analyte.

12. Procédé de correction d'un signal généré par un analyte, comprenant les étapes consistant en:
(a) l'introduction d'un élément de diagnostic selon l'une des revendications 1 à 9 dans un instrument de mesure analytique,
(b) la génération d'un premier signal détectable dans l'instrument de mesure analytique en mettant en contact l'analyte avec l'élément de diagnostic,
(c) la génération d'un second signal détectable dans l'instrument d'analyse par libération de l'analyte indicateur dans l'élément de diagnostic, et
(d) la présentation du signal généré dans l'étape (b) en utilisant le signal généré dans l'étape (c).

13. Procédé de contrôle de l'optique de détection d'un instrument de mesure analytique, comprenant les étapes consistant en:
(a) l'introduction d'un élément de diagnostic selon l'une des revendications 1 à 9 dans l'instrument de mesure analytique,
(b) la génération d'un signal détectable dans l'instrument de mesure analytique par libération de l'analyte indicateur dans l'élément de diagnostic, et
(c) la mise en corrélation du signal produit à l'étape (b) avec un signal de référence.

14. Système pour la libération contrôlée d'un réactif pour la détermination spécifique d'un analyte comprenant
(a) un support comprenant le réactif et un système de réaction indépendant de celui-ci, dans lequel le réactif est présent sous une forme libérable inaccessible pour une réaction avec le système de réaction, et peut être libéré dans des conditions définies,
(b) des moyens pour libérer le réactif sur le support.

15. Système selon la revendication 14,
**caractérisé en ce**
**que** le réactif est encapsulé dans une matrice soluble, est séparé du système de réaction par une couche barrière soluble, ou/et comprend un groupement protecteur chimique.

16. Utilisation d'un système selon la revendication 14 ou 15 comme élément de commutation.


### SEQUENCE LISTING

<110> F. Hoffmann - La Roche AG Roche Diagnostics GmbH
<120> Detektion der Zersetzung von Enzymen in einem Testelement durch kontrollierte Freisetzung von geschütztem Analyt
<130> 43826P WO
<150> EP 09 179 500.5
   <151> 2009-12-16
<160> 2
<170> PatentIn version 3.3
<210> 1
   <211> 261
   <212> PRT
   <213> Artificial
<220>
   <223> Mutant of glucose dehydrogenase from Bacillus subtilis
<220>
   <221> MUTAGEN
   <222> (96)..(96)
<220>
   <221> MUTAGEN
   <222> (170)..(170)
<400> 1
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Gly
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
               115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Lys Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
           195                 200                 205
   Val Glu Ser Met Ile Pro Met Gly Tyr Ile Gly Glu Pro Glu Glu Ile
       210                 215                 220
   Ala Ala Val Ala Val Trp Leu Ala Ser Lys Glu Ser Ser Tyr Val Thr
   225                 230                 235                 240
   Gly Ile Thr Leu Phe Ala Asp Gly Gly Met Thr Lys Tyr Pro Ser Phe
                   245                 250                 255
   Gln Ala Gly Arg Gly
               260
<210> 2
   <211> 261
   <212> PRT
   <213> Artificial
<220>
   <223> Mutant of glucose dehydrogenase from Bacillus subtilis
<220>
   <221> MUTAGEN
   <222> (170)..(170)
<220>
   <221> MUTAGEN
   <222> (252)..(252)
<400> 2
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
   35 40 45
   Lys Glu Glu Val Ile Lys Ala Gly Gly Glu Ala Val Val Val Gln Gly
   50 55 60
   Asp Val Thr Lys Glu Glu Asp Val Lys Asn Ile Val Gln Thr Ala Ile
   65 70 75 80
   Lys Glu Phe Gly Thr Leu Asp Ile Met Ile Asn Asn Ala Gly Leu Glu
   85 90 95
   Asn Pro Val Pro Ser His Glu Met Pro Leu Lys Asp Trp Asp Lys Val
   100 105 110
   Ile Gly Thr Asn Leu Thr Gly Ala Phe Leu Gly Ser Arg Glu Ala Ile
   115 120 125
   Lys Tyr Phe Val Glu Asn Asp Ile Lys Gly Asn Val Ile Asn Met Ser
   130 135 140
   Ser Val His Glu Val Ile Pro Trp Pro Leu Phe Val His Tyr Ala Ala
   145 150 155 160
   Ser Lys Gly Gly Ile Lys Leu Met Thr Lys Thr Leu Ala Leu Glu Tyr
   165 170 175
   Ala Pro Lys Gly Ile Arg Val Asn Asn Ile Gly Pro Gly Ala Ile Asn
   180 185 190
   Thr Pro Ile Asn Ala Glu Lys Phe Ala Asp Pro Lys Gln Lys Ala Asp
   195 200 205
   Val Glu Ser Met Ile Pro Met Gly Tyr Ile Gly Glu Pro Glu Glu Ile
   210 215 220
   Ala Ala Val Ala Val Trp Leu Ala Ser Lys Glu Ser Ser Tyr Val Thr
   225 230 235 240
   Gly Ile Thr Leu Phe Ala Asp Gly Gly Met Thr Leu Tyr Pro Ser Phe
   245 250 255
   Gln Ala Gly Arg Gly
   260